# NEW EUROPEAN PATENT SPECIFICATION

(11) **EP 2 551 265 B2**
(45) Date of publication and mention of the opposition decision: **13.09.2017**
(45) Mention of the grant of the patent: 07.01.2015
(21) Application number: 12169743.7
(22) Date of filing: 29.05.2012
(51) Int. Cl.: C07D 213/50, C07C 317/32

(54) **Novel process for the preparation of 1-(6-methylpyridin-3-yl)-2-[4-(methylsulfonyl)phenyl]ethanone, an intermediate of etoricoxib.**
Neues Verfahren zur Herstellung von 1-(6-Methylpyridin-3-yl)-2-[4-methylsulfonyl)phenyl]ethanon, ein Zwischenstoff von Etoricoxib
Nouveau procédé de préparation de 1-(6-méthylpyridine-3-yl)-2-[4-(méthylsulfonyl)phényl] éthanone, un intermédiaire d'étoricoxib

(30) Priority: 29.07.2011 IT MI20111455
(43) Date of publication of application: 30.01.2013
(73) Proprietor: F.I.S.- Fabbrica Italiana Sintetici S.p.A., 36075 Alte di Montecchio Maggiore (VI) (IT)
(72) Inventor: Brasola, Elena, I-35030 Saccolongo, Padova (IT); Fontana, Francesco, I-22030 Longone al Segrino, Como (IT); Stabile, Paolo, I-37100 Verona (IT); Galvagni, Marco, I-37131 Verona (IT)
(74) Representative: Croce, Valeria

(56) References cited:
- DAVIES I W ET AL: "A PRACTICAL SYNTHESIS OF A COX-2-SPECIFIC INHIBITOR", JOURNAL OF ORGANIC CHEMISTRY, ACS, US, vol. 65, 1 January 2000 (2000-01-01), pages 8415-8420, XP002326845, ISSN: 0022-3263, DOI: 10.1021/JO000870Z

## Description

### Technical field of the invention

A process for the preparation of 1-(6-methylpyridin-3-yl)-2-[4-(methylsulfonyl)phenyl]ethanone, an intermediate of the synthesis of Etoricoxib, which is a pharmaceutical anti-inflammatory active ingredient belonging to the class of the COX-2 inhibitors forms an object of the present invention.

### State of the art

The 1-(6-Methylpyridin-3-yl)-2-[4-(methylsulfonyl)phenyl] ethanone of formula (I): 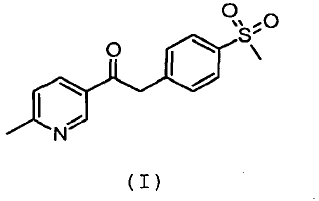 having CAS RN 221615-75-4 is an important intermediate for the synthesis of Etoricoxib which is a pharmaceutical active ingredient having anti-inflammatory activity being part of the class of the COX-2 inhibitors and it has been available in the market, in suitable pharmaceutical form, since 2002 under the commercial name of Arcoxia.

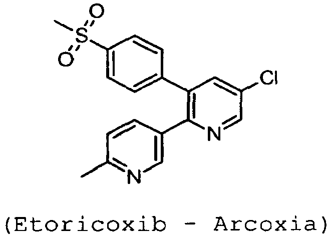

Examples of the use of such compound for the synthesis of COX-2 inhibitors are indicated in WO9955830, WO9915503 and by Davies, Lan W et al. in Journal of Organic Chemistry (2000), 65(25), 8415-8420.

Several methods for the synthesis of such an important building block are known, besides those described in the aforementioned patent documents, the method described in WO 2001/007410 by Lonza and Merck & Co., which consists of at least three synthetic steps, seems to be the most economically advantageous one. A disadvantage of such process lies in the fact that it involves, as the last step, an oxidation with hydrogen peroxide catalysed by sodium tungstate. The subsequent application WO 2001/029004 by Zambon Group S.p.A. describes an improved process for carrying out the aforementioned oxidation, which involves the combination of an oxidant (for example, a mixture of peracetic acid and hydrogen peroxide) in the presence of a catalyst (sodium tungstate) and an acid (for example, methanesulfonic acid).

In a yet different process described by Davies Lan W et al. in Journal of Organic Chemistry (2000), 65(25), 8415-8420 it is stated that the reaction between the magnesium dianion of [(4-methylsulfonyl)phenyl] acetic acid of formula (IV): 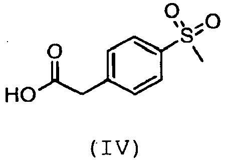 i.e. the compound of formula (V): 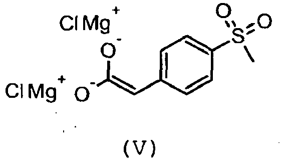 and/or (VI): 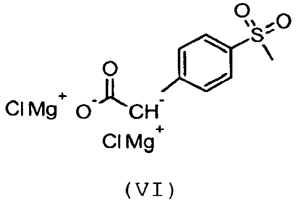 with the methyl ester of 6-methylpyridine-3-carboxylic acid of formula (III - R=Me): 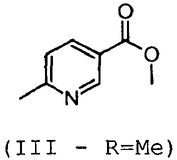 allows the preparation of 1-(6-methylpyridin-3-yl)-2-[4-(methylsulfonyl)phenyl]ethanone of formula (I): 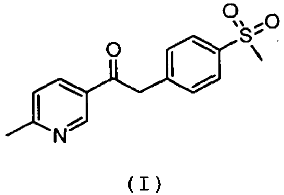 with conversion (not molar yield) below 50% employing 2 equivalents of tert-butylmagnesium chloride. However, the authors outline that the problem was resolved, by increasing the molar yield up to 58% by adding an extra molar equivalent of t-butylmagnesium chloride during the reaction, thus generally employing 3 equivalents of Grignard reagent instead of the two typically employed for this kind of reactions.

The experiment carried out in our laboratories however revealed that repeating such process i.e. using 3 equivalents of Grignard reagent with the same substrates, whereas it is true that the reaction goes to completion obtaining a product with similar yields, it is observed that the product contains unacceptable amounts, of the order of 3%, of an impurity having a molecular weight equivalent to 408.5 according to LC/MS determination. Also according to other experimental observations, the subsequent preparation of an enriched sample followed by the preparative HPLC isolation and 1H-NMR and 13C-NMR spectrometry allowed to identify such impurity which thus has the following structural formula (XII): 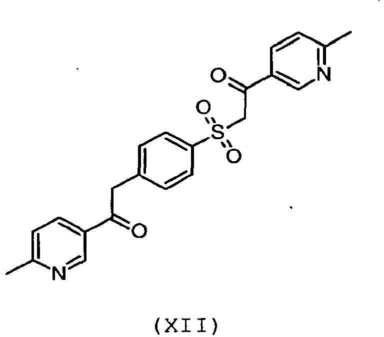 to which the IUPAC name of 1-(6-methylpyridin-3-yl)-2-({4-[2-(6-methylpyridin-3-yl)-2-oxoethyl]phenyl}sulfonyl)ethanone corresponds.

Such process impurity (also referred to as '408') resulted to be particularly difficult to be removed from the product of formula (I), unless to the detriment of consistent yield losses due to various required recrystallisations of the product. Also the addition - in 3 portions - of Grignard reagent alternated with 3 portions of the methyl ester of 6-methylpyridin-3-carboxylic acid, intervalled with respect to each other, leads to a product of formula (I) containing 2 to 4 percent of impurity '408' (see the comparative example 6).

Lastly, starting from the methyl ester of (4-methylsulfonyl)phenyl acetic acid of formula (IV) instead, Davies Lan W et al. - in Journal of Organic Chemistry (2000), 65(25), 8415-8420 - argue that there are attained only small amounts of product of formula (I).

### Summary of the invention

The problem addressed by the present invention is thus that of providing an improved process for the preparation of 1-(6-methylpyridin-3-yl)-2-[4-(methylsulfonyl)phenyl]ethanone of formula (I) which allows obtaining the product in high yields and simultaneously limiting the formation of the impurity "408".

Such problem is resolved by a process for the preparation of 1-(6-methylpyridin-3-yl)-2-[4-(methylsulfonyl)phenyl]ethanone as outlined in the attached claims, whose definitions are an integral part of the present description.

Further characteristics and advantages of the process according to the invention will be apparent from the description indicated below of the preferred embodiment, provided by way of non-limiting example.

### Brief description of the figures:

By way of example:
Figure 1 shows a 1H-NMR (400 MHz, D₂O) spectrum of (4-methylsulfonyl)phenyl acetate sodium;

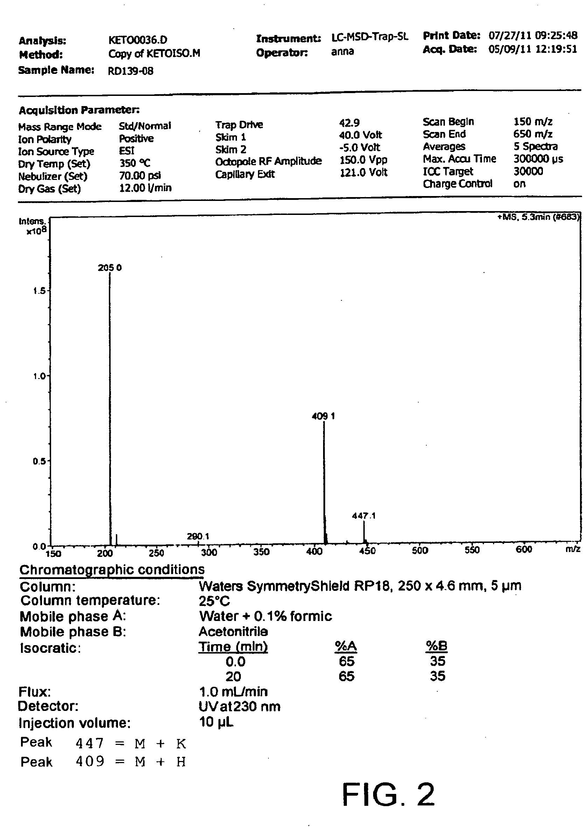

Figure 2 shows an LC/MS spectrum of the impurity of formula (XII);
Figure 3 shows a HPLC chromatogram acquired through the HPLC method of Example 8 regarding a product of formula (I) obtained according to Example 3 according to the invention;
Figure 4 shows a HPLC chromatogram acquired through the method of Example 8 regarding a product of formula (I) obtained according to Example 6 (comparative of the invention);
Figure 5 shows the experimental results according to the invention and through the comparative experiments;

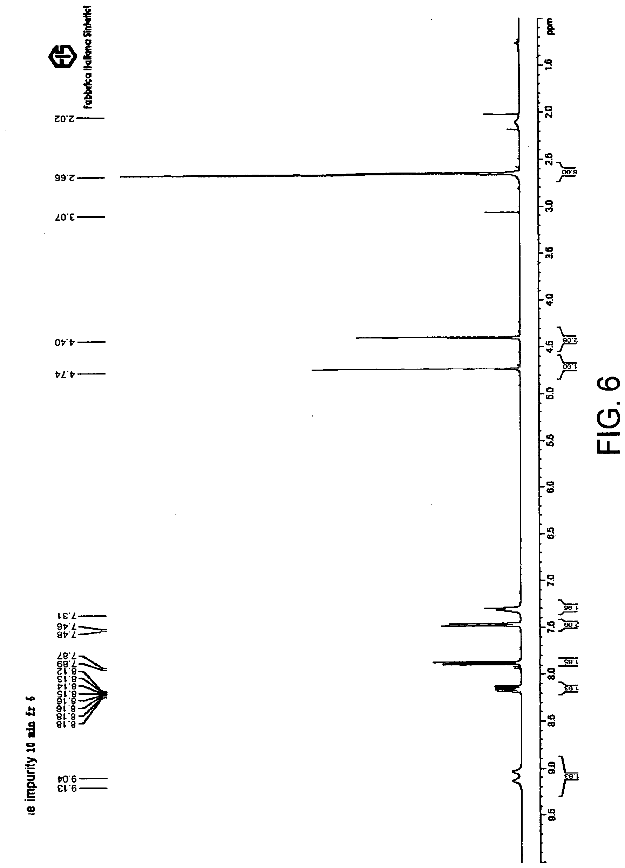

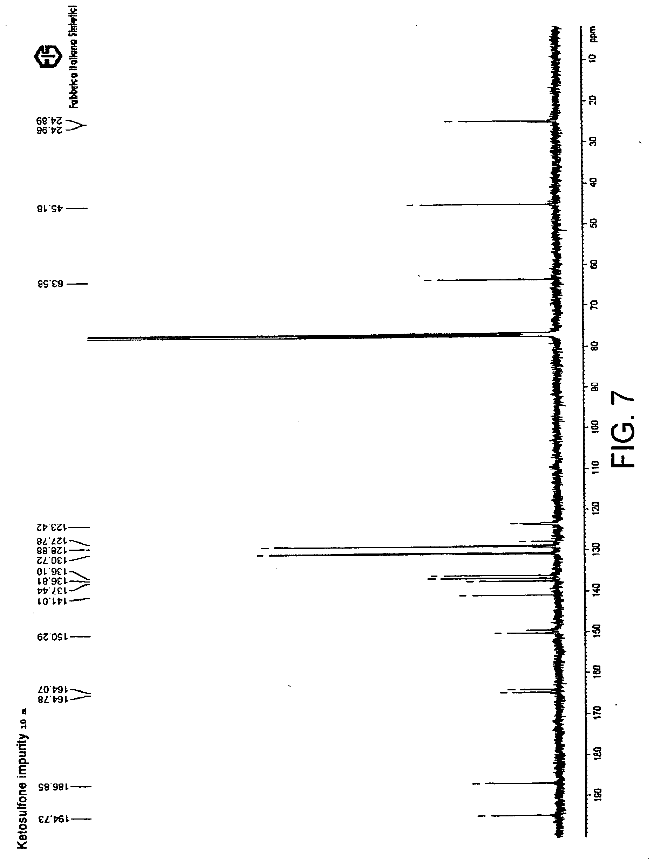

Figures 6 and 7 show the 1H-NMR and the C13-NMR spectra of compound '408' having formula (XII), respectively.

### Detailed description of the invention

The present invention regards a process for the preparation of 1-(6-methylpyridin-3-yl)-2-[4-(methylsulfonyl)phe-nyl]ethanone of formula (I) or a salt thereof: 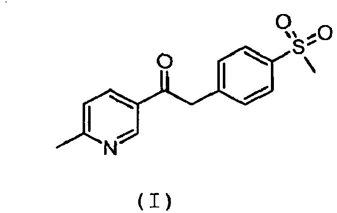 comprising the reaction of (4-methylsulfonyl)phenyl acetic acid of formula (IV): 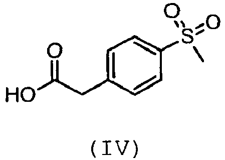 or an alkaline salt of (4-methylsulfonyl) phenyl acetic acid of formula (II): 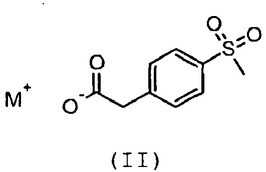 wherein M⁺ is an ion of an alkaline metal,
with the ester of 6-methylpyridine-3-carboxylic acid of formula (III): 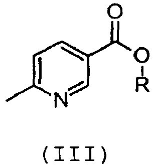 wherein R is a C1-C5 linear or branched alkyl or an aryl or a substituted aryl, a benzyl or a substituted benzyl, characterised in that the addition of the two reagents such as the ester of the 6-methylpyridine-3-carboxylic acid of formula (III) and the Grignard reagent to the (4-methylsulfonyl)phenyl acetic acid of formula (IV) or to the alkaline salt of the (4-methylsulfonyl)phenyl acetic acid of formula (II) is carried out simultaneously.

The simultaneous addition of the two reagents i.e. of the ester of 6-methylpyridine-3-carboxylic acid of formula (III) and the Grignard reagent is intended to be a concurrent addition. The addition may be carried out both by adding the reagents in solid form rather than by adding solutions comprising them. The two reagents i.e. the ester of 6-methylpyridine-3-carboxylic acid of formula (III) and the Grignard reagent are added separately i.e. it is not intended that they are previously mixed together but they are added simultaneously to (4-methylsulfonyl)phenyl acetic acid of formula (IV) or to the alkaline salt of (4-methylsulfonyl) phenyl acetic acid of formula (II). The addition of the two reagents is thus usually carried out as a simultaneous dosage thereof.

The simultaneous or concurrent addition or dosage of the two reagents can be carried out continuously or discontinuously, for example providing for breaks between the various simultaneous dosages. This is the case of continuous or simultaneous addition or dosage of discrete amounts of the two reagents. Best results, in terms of impurity '408' are however obtained by performing the simultaneous addition of the two reagents continuously. The sequential addition of discrete amounts of the two reagents obtained discontinuously leads to markedly poorer results; actually, performing for example 3 additions of Grignard reagent each of which is immediately followed by the addition of the ester of the 6-methylpyridine-3-carboxylic acid of formula (III) then waiting, leads to markedly poorer results in terms of impurity '408', whose content may reach up to 3%.

The sequential addition of discrete amounts of the two reagents performed continuously (i.e. one first, then the other, one first, then the other, etc; without breaks) such to be carried out in more than 6 sequential additions of each reagent performed continuously (i.e. without breaks between the continuous additions) should be deemed comparable to a simultaneous addition of the two reagents and thus comparable to the continuous additions of the present invention. Best results are obtained however by adding the two reagents simultaneously and continuously rather than adding the two reagents sequentially and continuously by at least 6 additions of each reagent.

The alkaline salt of (4-methylsulfonyl) phenyl acetic acid of formula (II) may be a Lithium, Sodium, Potassium, Rubidium, Cesium and Francium salt. According to a particularly preferred aspect the best process is carried out using the lithium salt of (4-methylsulfonyl)phenyl acetic acid. Also (4-methylsulfonyl)phenyl acetic acid of formula (IV) provides good results employing the process of the invention.

The ester of 6-methylpyridine-3-carboxylic acid of formula (III) is a C1-C5 linear or branched alkyl ester or an aryl or a substituted aryl, a benzyl or a substituted benzyl. R may thus be methyl, ethyl, propyl, n-propyl, isopropropyl, butyl, n-butyl, isobutyl, sec-butyl, ter-butyl, pentyl, n-pentyl, 4-methylbutyl, 3-ethylpropyl, 3,3-dimethylpropyl or phenyl, benzyl, substituted phenyl or substituted benzyl. According to a preferred aspect, R is methyl or ethyl.

The preparation of the alkaline salt of (4-methylsulfonyl)phenyl acetic acid of formula (II) may be performed by means of a basic salt of an alkaline metal, preferably selected from among the group consisting in hydroxide, hydride, C1-C5 alkoxy. The (4-methylsulfonyl) phenyl acetic acid of formula (II) is thus reacted with sodium hydroxide, potassium hydroxide or lithium hydroxide or with sodium methoxide, sodium ethoxide, potassium methoxide or potassium ethoxide, sodium hydride or potassium hydride. Preferably, the preparation of said salt is carried out with the hydroxide of an alkaline metal such as sodium hydroxide, potassium hydroxide or lithium hydroxide.

Furthermore, the reaction is carried out in an organic solvent, preferably in methanol or THF.

The addition reaction of (4-methylsulfonyl)phenyl acetic acid of formula (IV) or the alkaline salt of (4-methylsulfonyl)phenyl acetic acid of formula (II) on the ester of 6-methylpyridine-3-carboxylic acid of formula (III) is carried out by means of a C1-C5 alkyl Grignard reagent. Thus, there may be used Methylmagnesium chloride or bromide, Ethylmagnesium chloride or bromide, isopropylmagnesium chloride or bromide, tert-butylmagnesium chloride. Preferably, the reaction is carried out using tert-butylmagnesium chloride.

In case of use of an alkaline salt of (4-methylsulfonyl)phenyl acetic acid of formula (II), the addition reaction first occurs with the formation of the mixed dianion of formula (VII): 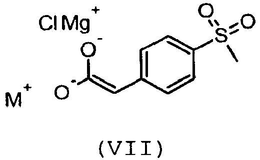 and/or of formula (VIII): 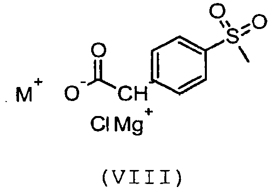 and/or of formula (IX): 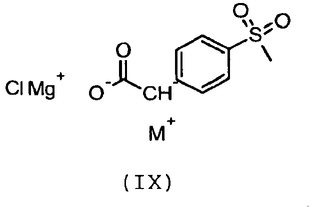 wherein M+, in the compound of formula (VII), (VIII) and (IX), is an ion of an alkaline metal and, subsequently, there occurs the addition thereof on the ester of the 6-methylpyridine-3-carboxylic acid of formula (III): 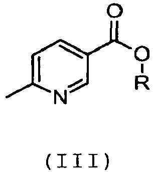 to give the transient compound of formula (X): 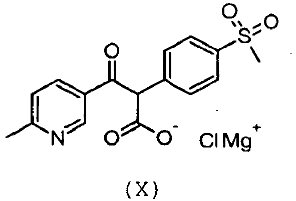 and/or of formula (XI):

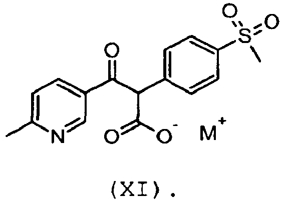

This/these compound/s decarboxylate/s to give the compound of the invention of formula (I).

Given that these are ionic species, transient at times and balanced with respect to each other, the composition of the actual chemical species involved was not studied further; however the presence of the ion of an alkaline metal instead of the Magnesium monochloride (MgCl) ion provides a positive effect on the yield of the reaction, due to the better addition between the two synthons and/or the better decarboxylation of the obtained transient intermediates. Furthermore, another effect that is observed, especially in the case wherein M is lithium, lies in the fact that there occurs a reduction of the amount of impurity of formula '408' formed in the reaction, though maintaining high yields of the product of formula (I).

The addition reaction is carried out in an organic solvent, preferably in THF.

The reaction is carried out by using 10 to 30 volumes of solvent, preferably it is carried out in about 20 volumes of solvent.

Should the reaction be carried out using (4-methylsulfonyl)phenyl acetic acid of formula (IV) there are used from 2.0 to 4.0 molar equivalents of Grignard reagent, preferably there are used about 3.0 equivalents.

Should the reaction be carried out using the alkaline salt of (4-methylsulfonyl)phenyl acetic acid of formula (II) there are used from 1.5 to 3.0 molar equivalents of Grignard reagent, preferably there are used from 1.6 to 1.8 molar equivalents of reagent.

The reaction is carried out between 40° and 70°C, preferably it is carried out at 65°C.

The simultaneous dosage of the Grignard reagent and of 6-methylpyridine-3-carboxylic acid of formula (III) ester is carried out, for preparations at laboratory level, over a period of time comprised between 30 minutes and 2 hours, preferably it is carried out in 60 minutes.

The product is then isolated using the conventional organic synthesis techniques comprising extractions and crystallizations.

The typical molar yield of the process according to the present invention is comprised in the range between 78%-88% molar.

The process for the preparation of 1-(6-methylpyridin-3-yl)-2-[4-(methylsulfonyl)phenyl]ethanone according to the present invention has the advantage of avoiding the use of tungsten-based catalyst. A further advantage lies in that the process of the present invention uses raw materials of formula (II) and (III) widely available in the market (when R=Me, see Lonza AG). The raw material (4-methylsulfonyl)phenyl acetic acid of formula (IV) having CAS RN 90536-66-6 is actually a substance widely available in the market.

Furthermore, the raw material of formula (II or IV) has sulphur already in the oxidation state required by the final product, which allows avoiding the final oxidation thus, with respect to the processes of the prior art, it allows avoiding having to use hydrogen peroxide and organic peroxides which are potentially hazardous substances due to explosiveness. Furthermore, such oxidizing reagents typically lead to the Pyridine N-Oxide reaction by-product as described in Journal of Organic Chemistry (2000), 65(25), 8415-8420. Furthermore, a further advantage of the process according to the present invention lies in that the described synthetic path converges with respect to the state of the art. In terms of industrial applicability the convergence of the process allows isolating the effects of critical process parameters from the quality of the final product.

According to an embodiment, the alkaline salt of (4-methylsulfonyl)phenyl acetic acid of formula (II), prepared following the conversion of (4-methylsulfonyl)phenyl acetic acid of formula (IV): 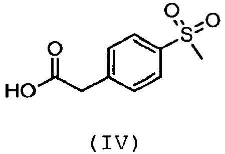 in the corresponding alkaline salt of formula (II): 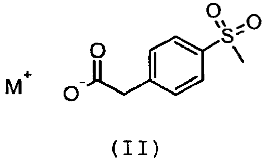 wherein M⁺ is an ion of an alkaline metal; may be conveniently isolated or the preparation of said salt and the subsequent addition reaction to the ester of 6-methylpyridine-3-carboxylic acid of formula (III) may be carried out consecutively without the isolation of the alkaline metal salt of (4-methylsulfonyl)phenyl acetic acid of formula (II), allowing to obtain the product of formula (I) *one-pot* i.e. with only one synthetic step thus avoiding at least three synthetic steps with respect to other known processes. Thus, the process of the present invention is extremely advantageous from an economical point of view.

Still another advantage of the present process is, in case of use of the salts of formula (II), the use of only 1.6-1.8 molar equivalents of Grignard reagent instead of the 3 required for by the known improved processes with evident greater economical advantages of the process.

Lastly, the process of the present invention allows the recovering and the recycling of (4-methylsulfonyl)phenyl acetic acid reagent or the alkaline metal salt of the (4-methylsulfonyl)phenyl acetic acid of formula (II) used as the excess reagent in the synthesis.

The 1-(6-methylpyridin-3-yl)-2-[4-(methylsulfonyl)phenyl] ethanone of formula (I) obtained with the process according to the present invention comprises the impurities of formula (XII) in a range comprised between 0.03 and 0.6 percent and preferably between 0.10 and 0.40 percent. Though recrystallizing once the product obtained with the process of the invention from 8 volumes of Acetonitrile/Acetone mixture (1:1) it was not possible to reduce the amount of such impurity below 0.03% (HPLC, Area percent) and thus this impurity, not present in the compound (I) prepared through other processes starting from different raw materials, results to be a marker of the product obtained through the process of the present invention. In particular, all processes of the prior art starting from the same raw materials of the process of the present invention lead to a product of formula (I) comprising between 2% and 4% HPLC A% of such impurity. Actually, the product of formula (I) obtained through the Lonza/Merck industrial process acccording to WO 2001/007410 does not comprise such impurity of formula (XII) and in addition, the processes of the prior art, like the one described in Journal of Organic Chemistry (2000), 65(25), 8415-8420, lead to a product of formula (I) comprising more than 2% of such impurity. Though the removal of this impurity of formula (XII) is essential for the product since it was experimentally observed that it is a precursor of impurities that are difficult to remove from the Etoricoxib product, there is no evidence that the removal of such impurity from the compound of formula (I) has ever been sought in the prior art.

The impurity of formula (XII), which can be prepared for example by preparing an enriched sample thereof i.e. following the prior art process described on the Journal of Organic Chemistry mentioned above, and isolating it - through preparative HPLC - it can be conveniently used as a Reference Standard or Reference Marker for determining the content of such impurity in the compound of formula (I). Such determination can be carried out by means of chromatographic methods and in particular by means of HPLC, GC or TLC; preferably by means of HPLC.

The 1-(6-methylpyridin-3-yl)-2-[4-(methylsulfonyl)phenyl]ethanone of formula (I) obtained through the process according to the present invention may thus be conveniently used for the preparation of the Etoricoxib active ingredient (according to the preparations indicated on the aforementined Journal of Organic Chemistry) which, formulated pharmaceutically, is used as a drug and in particular it is used as a nonsteroidal anti-inflammatory drug (NSAID) and, even more specifically, as a COX-2 inhibitor (COXIB).

### EXPERIMENTAL PART

### Example 1 - Synthesis of (4-methylsulfonyl) phenyl acetate sodium of formula (II - M=Na) - example of the invention.

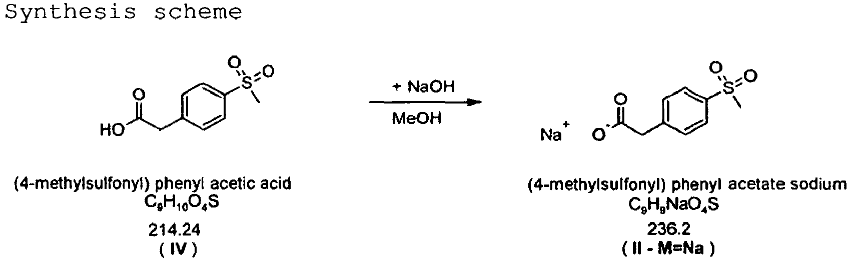

In a 4-neck flask there are introduced at 20-25°C 25 g of (4-methylsulfonyl)phenyl acetic acid of formula (IV) and 125 mL of Methanol. The mixture is heated up to 35-40°C and stirred for about 5-10 minutes up to obtaining complete solubilisation.

A solution of 5.6 g of NaOH in form of pearls (1.2 molar equivalents) in 25 mL of Methanol is prepared separately.

The sodium hydroxide solution in methanol is added in 1 h at 35-40°C into the acid sulfone solution. After the addition, the pH is about 14 (litmus paper).

It is left under stirring for 30 minutes then it is cooled to T= 0-5°C and it is left under stirring up to complete precipitation. The suspension is filtered and the solid is washed using cold methanol.

The product is dried at 60°C under vacuum for 8 hrs to obtain 22 g of product as a white solid for a molar yield equivalent to 80%.

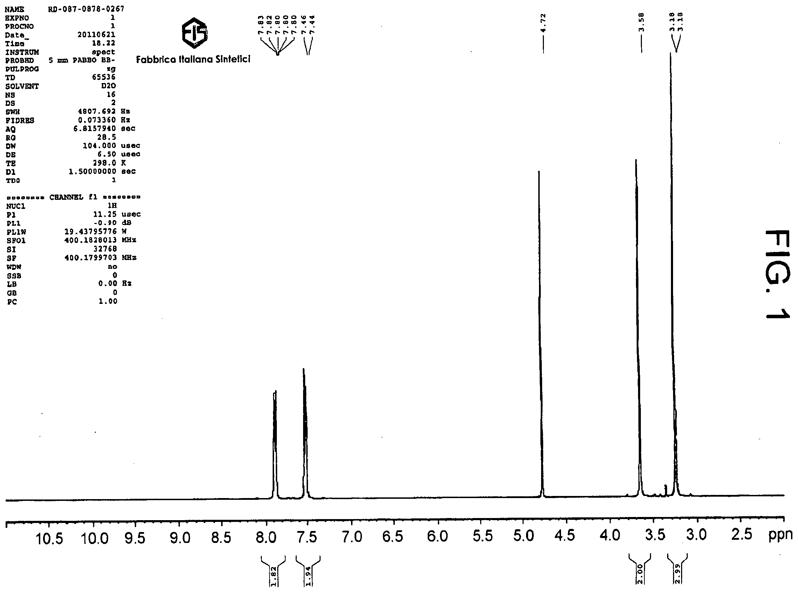

1H-NMR (Figure 1): 3.26 (s, 3H, CH3); 3.58 (s, 2H, CH2); 7.45 (d,J=8 Hz,2H, Ar); 7.80 (d,J=8 Hz,2H, Ar); Melting point = 275.4°C (determined through DSC); HPLC purity = 99.82 (A%). K.F. =0.13%.

### Example 2 - Synthesis of (4-methylsulfonyl) phenyl acetate lithium of formula (II - M=Li) - example of the invention according to a preferred aspect.

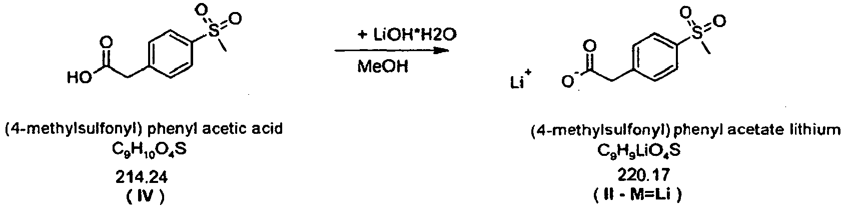

In a 4-neck flask there are introduced at 20-25°C 150 g of (4-methylsulfonyl)phenyl acetic acid of formula (IV) (1.0 mol. equiv.) and 750 mL of Methanol. Stirring is carried out at 35-40°C up to obtaining complete solubilisation.

32 g of LiOH monohydrate (1.08 molar equivalents) are added maintaining at T=35/40°C. The precipitation of the lithium salt occurred.

Stirring is carried out at 35-40°C for one hour then it is cooled to room temperature and stirring is carried out for 2 hours.

The suspension is filtered and the solid is washed using Methanol. The solid is dried under vacuum at T=90°C for 8 hours to afford 136 g of product as a white solid for a molar yield equivalent to 88%.

1H-NMR: 3.26 (s, 3H, CH3); 3.65 (s, 2H, CH2); 7.45 (d,J=8 Hz,2H, Ar); 7.89 (d,J=8 Hz,2H, Ar); Melting point = 334°C (determined through DSC); HPLC purity = 99,51 (A%). K.F. = 0.11%.

### Example 3 - 1-(6-methylpyridin-3-yl)-2-[4-(methylsulfonyl)phenyl]ethanone of formula (I) - example of the invention according to a preferred aspect.

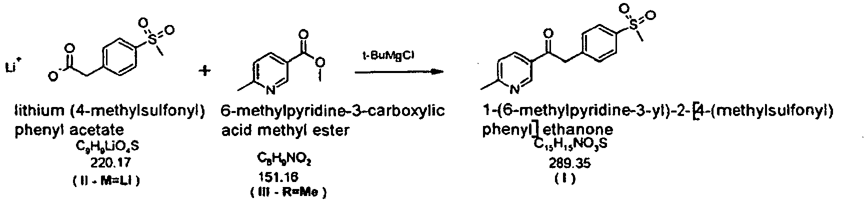

In a 4-neck anhydrous flask there are introduced at 20-25°C, 10.2 g of Lithium (4-methylsulfonyl)phenyl acetate of formula (II- M=Li) (1.0 mol. equiv), 200 mL of Anhydrous THF and the mixture is heated to 65-70°C (up to reflux).

Maintaining at T=65-70 °C there are simultaneously dosed in the mixture in about 1 hour:
a) 66.0 g of t-BuMgCl 1.0 M solution in THF (about 74.2 mL) (1.6 mol. equiv.) and
b) a solution of 4.64 g of the methyl ester of the 6-methylpyridine-3-carboxylic acid of formula (III - R=Me) (0.65 mol. equiv.) in 15 mL of Anhydrous THF.

After completing the addition, the mixture is stirred at 65-70 °C for 30 minutes.

The reaction is controlled through HPLC then it is cooled to 20-25°C and the reaction mixture is diluted under vigorous stirring with 100 mL of water. Maintaining the temperature comprised in the range between 20°C and 25°C the mixture is brought to pH comprised between 1 and 0 by adding about 15 mL of 32% hydrochloric acid.

Stirring is carried out at 20-25°C for 30 minutes controlling the pH.

The phases are separated by putting aside the organic phase useful for recovering the lithium (4-methylsulfonyl)phenyl acetate.

The aqueous phase is washed using 2x50 mL of MTBE and the organic phases are recombined and used for recovering the (4-methylsulfonyl)phenyl acetate lithium. The aqueous phase is distilled at low pressure for removing the residue organic solvent. The product is precipitated under heat (40-45°C) by adding 7.5 mL of 30% aqueous NaOH followed by cooling to room temperature. The product is filtered and dried in an oven under vacuum at 50°C for 8 hours.

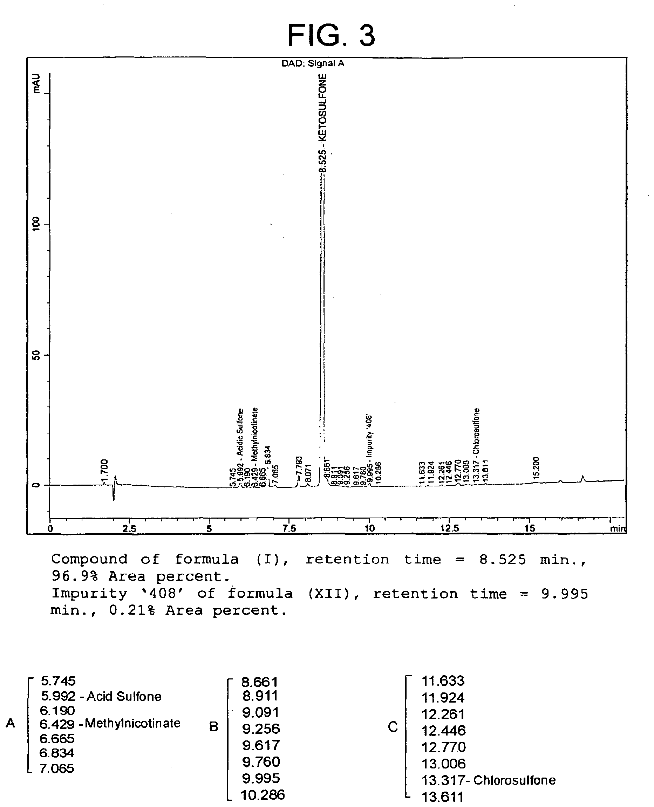

There are obtained 6.9 g of product as a white solid corresponding to a 78% molar yield. HPLC purity 96.9% (A%). Impurity '408' = 0.21% (HPLC A%) (Figure 3).

The organic phases obtained as described above are recombined to bring to a pH comprised between 11 and 12 by adding about 1 g of LiOH monohydrate solid. The mixture is concentrated to residue at low pressure at a maximum temperature of 40°C. The residue is suspended at room temperature in 2 volumes of Methanol. Stirring is carried out at room temperature for at least 3 hours. The suspension is filtered and the solid is washed using cold methanol then dried at low pressure at 90°C for 8 hours, to obtain 2.5 g of (4-methylsulfonyl)phenyl acetate lithium.

### Example 4 - 1-(6-methylpyridin-3-yl)-2-[4-(methylsulfonyl)phenyl]ethanone of formula (I) - example of the invention.

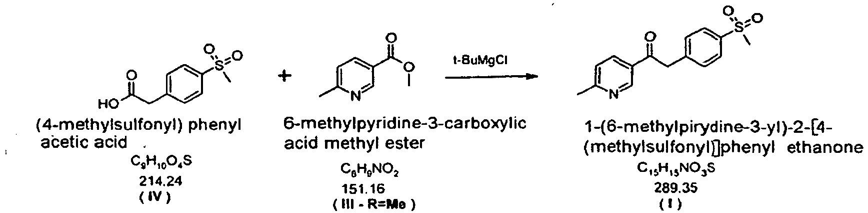

In a 4-neck anhydrous flask there are introduced at 20-25°C 5.0 g of (4-methylsulfonyl)phenyl acetic acid of formula (IV) (1.0 mol. equiv.), 100 mL of Anhydrous THF and the mixture is heated to 65-70°C (up to reflux).

Maintaining at T=65-70 °C there are simultaneously dosed in the mixture in about 1 hour:
a) 62.0 g of t-BuMgCl 1.0 M solution in THF (about 70.0 mL) (3.0 mol. equiv.) and
b) a solution of 2.3 g of methyl ester of 6-methylpyridine-3-carboxylic acid of formula (III - R=Me) (0.65 mol. equiv.) in 7.5 mL of Anhydrous THF.

After completing addition, maintain at 65-70 °C for 30 minutes.

The reaction is controlled through HPLC: HPLC purity 72.05% (A%) and Impurity '408' = 0.26% (A%).

### Example 5 - 1-(6-methylpyridin-3-yl)-2-[4-(methylsulfonyl)phenyl]ethanone of formula (I) - comparative of the invention - 3 discontinuous sequential additions.

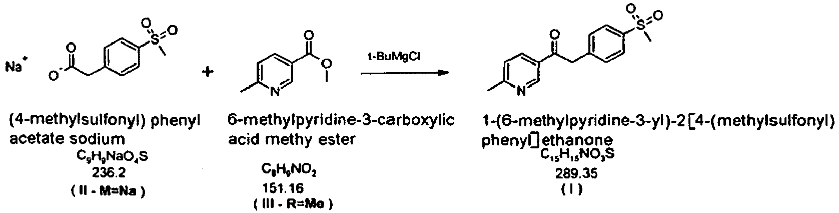

In a 4-neck anhydrous flask there are introduced at 20-25°C 11 g of (4-methylsulfonyl)phenyl acetate sodium of formula (II- M=Na), 200 mL of Anhydrous THF and the mixture is heated to 65-70°C.

Maintaining at T=65-70°C there are rapidly added 21 g (0.5 equiv.) of t-BuMgCl 1.0 M solution in THF (about 23 mL). The mixture is left under stirring for 1 hr at 65-70 °C then there is rapidly added, still at 65-70°C, a solution of 3.6 g of methyl ester of the 6-methylpyridine-3-carboxylic acid of formula (III - R=Me) (0.5 equiv.) in 10 mL of Anhydrous THF. After completing addition the mixture is maintained at 65-70 °C for 1 hr. Maintaining at T=65-70 °C there are rapidly added 21 g (0.5 equiv.) of t-BuMgCl 1.0 M solution in THF (about 23 mL). It is left under stirring for 1 hr at 65-70°C then there is rapidly added, still at 65-70°C, a solution of 520 mg of methyl ester of the 6-methylpyridine-3-carboxylic acid of formula (III - R=Me) (0.075 equiv.) in 2 mL of anhydrous THF.

After completing addition, the mixture is maintained at 65-70°C for 1 hr.

Maintaining at T=65-70 °C, there are rapidly added 33.6 g (0.8 equiv.) of t-BuMgCl 1.0 M solution in THF (about 37 mL).

The mixture is left under stirring for 1 hr at 65-70°C then there is rapidly added, still at 65-70°C, a solution of 520 mg of methyl ester of the 6-methylpyridine-3-carboxylic acid of formula (III -R=Me) (0.075 equiv.) in 2 mL of Anhydrous THF.

The mixture is left under stirring for 1 hr at 65-70°C then it is cooled to 20-25°C.

The molar yield in solution is equivalent to 90%.

After completing the reaction cooling is carried out at 20-25°C and the reaction mixture is diluted under vigorous stirring using 100 mL of Water.

Maintaining at T= 20-25 °C the mixture is brought to pH=1-0 by adding about 25 mL of 32% Hydrochloric acid. Stirring is carried out at 20-25°C for 0.5 hrs controlling the pH.

The organic phase is put aside (recovery of the sodium salt of the acidic sulfone).

The aqueous phase is washed using 2x50 mL of MTBE.

The organic phases are combined to the previous ones and they are put aside (recovery of the sodium salt of the acidic sulfon).

The process continues with the aqueous phase containing the product.

The aqueous phase is distilled to remove the residual organic solvent.

The pH of the aqueous phase is corrected up to about pH=6.5-7.5 using 25 mL of 30% NaOH.

There is observed the precipitation of the product. Stirring is carried out for at least one hour at 20-25°C. Filtration is carried out by washing the solid using 20 mL of purified water and 20 mL of THF.

The raw product is dried in an oven at 50°C for 8 hrs to obtain 7.7 g of product for a molar yield equivalent to 87%. HPLC purity 85.9% (A%) and Impurity '408'= 3.85% (A%).

The (4-methylsulfonyl)phenyl acetate of formula (II-M=Na) used in excess is recovered as follows. The organic phase obtained as described above is brought to pH=11-12 by dosing a solution of 1.25 g of NaOH in form of pearls or scales in 10 mL of Methanol.

The mixture is concentrated to residue at low pressure at Tmax=40°C.

The residue is suspended at room temperature using 10 mL of methanol. Stirring is carried out for at least 3 hrs at room temperature.

Filtration is carried out by washing using 5 mL of methanol.

The raw product is dried in an oven at 50°C for 8 hrs to afford 3.5 g of recovered product.

### Example 6 - One-pot synthesis of 1-(6-methylpyridin-3-yl)-2-[4-(methylsulfonyl)phenyl]ethanone of formula (I) - comparative of the invention - 3 discontinuous sequential additions.

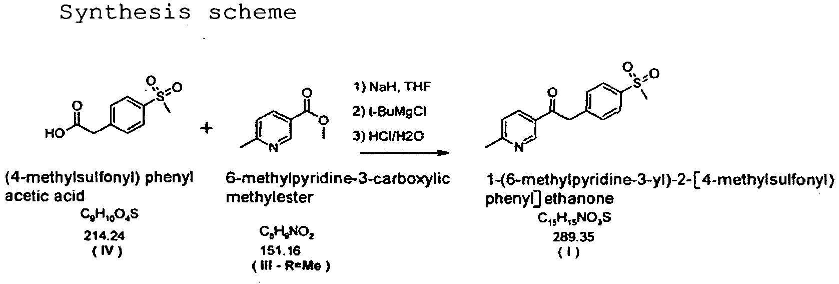

In an anhydrified 4-neck flask there were introduced at 20-25°C 5.0 g of (4-methylsulfonyl)phenyl acetic acid of formula (IV) and 100 mL of anhydrous THF and stirring is carried out at 20-25°C.

1.0 g of sodium hydride dispersion in mineral oil (60% w/w, MW: 24.00; 1.1 molar equivalents) is added in portions in about 5 minutes, ensuring that the temperature does not exceed 45-50°C.

After completing the addition the mixture is heated to 65-70°C for 1 hr. Maintaining at T=65-70°C there are rapidly added 21 g
(1.0 equiv.) of t-BuMgCl 1.0 M solution in THF (PM116.87) (23 mL). The mixture is left under stirring for 1 hr at 65-70°C then is rapidly added, still at 65-70°C, a solution of 1.78 g (0.5 equiv.) of methyl ester of the 6-methylpyridine-3-carboxylic acid of formula (III - R=Me) in 5 ml of Anhydrous THF. After completing the addition the mixture is maintained at 65-70°C for 1 hr.

Maintaining at T=65-70°C there are rapidly added 10.5 g (0.5 equiv.) of t-BuMgCl 1.0 M solution in THF (PM 116.87) (11.5 mL).

The mixture is left under stirring for 1 hr at 65-70°C then there is rapidly added, still at 65-70°C, a solution of 260 mg (0.075 equiv.) of methyl ester of 6-methylpyridine-3-carboxylic acid of formula (III - R=Me) in 1 mL of anhydrous THF. After completing addition the mixture is maintained at 65-70 °C for 1 hr.

Maintaining at T=65-70 °C there are rapidly added 10.5 g (0.5 equiv.) of t-BuMgCl 1.0 M solution in THF (PM 116.87) (11.5 mL).

The mixture is left under stirring for 1 hr at 65-70 °C then there is rapidly added, still at 65-70 °C, a solution of

260 mg (0.075 equiv.) of methyl ester of the 6-methylpyridine-3-carboxylic acid of formula (III - R=Me) in 1 mL of Anhydrous THF. After completing addition the mixture is maintained at 65-70 °C for 1 hr.

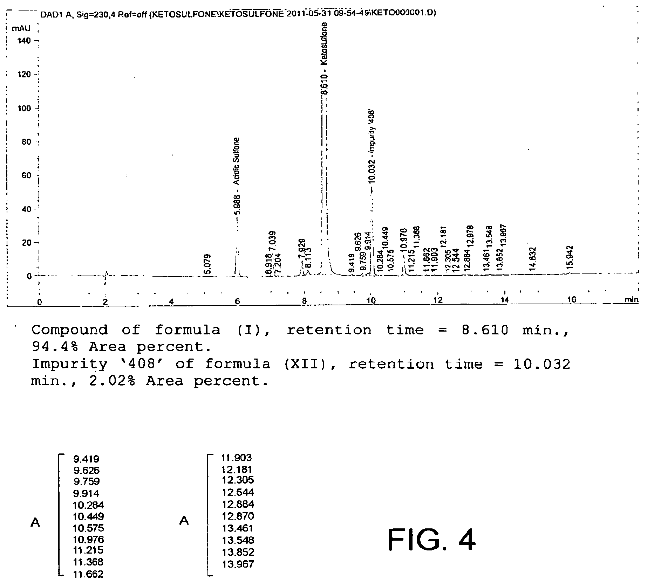

Cooling is carried out to 20-25°C. The yield is calculated in solution through titration: 80%. The product is isolated according to the preceding example. It is obtained a molar yield equivalent to 73% and with 94.4% HPLC purity (A%) and amount of impurity '408' = 2.02% (A%) (see figure 4).

### Example 7 - Synthesis of 1-(6-methylpyridin-3-yl)-2-[4-(methylsulfonyl)phenyl]ethanone of formula (I)-comparativetable.

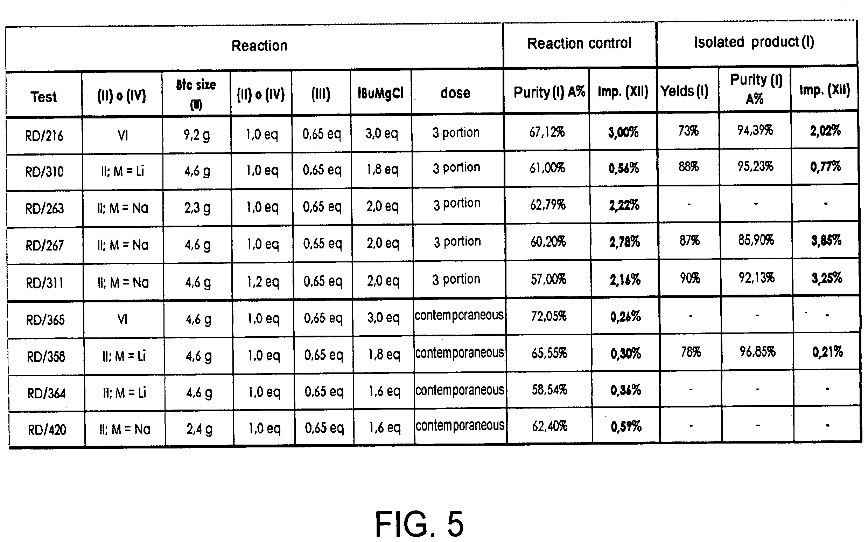

The tests summarized in the table of figure 5 were carried out. The reaction is carried out in all tests at T = 65°C and 20 volumes of solvent. The dosage of the reagents, for the tests where they were dosed simultaneously, was carried out in 1 hour. The first 5 tests were carried out using 3 non-simultaneous additions of the two reagents and thus they are comparative with respect to the invention.

### Example 8 - HPLC method for determining the content of the impurity of formula (XII) in the compound of formula (I):

### Chromatographic conditions:

| Column: | Waters SymmetryShield RP18, 150 x 4.6 mm, 3.5 µm | | |
|---|---|---|---|
| Column temperature: | 25°C | | |
| Mobile phase A: | Phosphate Buffer pH 3.5 | | |
| Mobile phase B: | Acetonitrile | | |
| Gradient: | Time (min) | %A | %B |
| | 0 | 90 | 10 |
| | 18 | 10 | 90 |
| Post-run time: | 5 minutes | | |
| Flow: | 1.0 mL/min | | |
| Detector: | UV 230 nm | | |
| Injection volume: | 5 µL | | |
| Auto-sampler: | 25°C | | |
| Duration of analysis: | 18 min | | |
| Diluent: | Water/ACN | 70:30 | |

The impurity of formula (XII) or impurity '408' has a relative retention time equivalent to 1.17 under these chromatographic conditions.

The quantitative determination is carried out in Area percent according to the usual methods known to chemical laboratory analysts.

The impurity '408' of formula (XII) can be isolated from the fractions of the eluate corresponding to the retention time of 1.17 (the chromatographic peak)

### Example 9 - Spectra of the impurity of formula (XII) or impurity '408':

1H NMR (400 MHz. CDCl3) δ: 9.13 (s, 1H); 9.04 (s, 1H); 8.15 (2dd, 2H); 7.88 (d, 2H, J=8.0 Hz);7.47 (d, 2H, J=8.0 Hz); 7.31 (m, 2H); 4.74 (s, 2H, -CH2); 4.40 (s, 2H, -CH2); 2.65 (s, 6H, 2 CH3).

13C NMR (100 MHz, CDCl3): 194.73, 186.85, 164.78, 164.07, 150.29, 141.01 ,137.44, 136.81, 136.10. 130.72, 128.88, 127.78, 123.42, 63.58, 45.18, 24.96, 24.89.

## Claims

1. Process for the preparation of 1-(6-methylpyridin-3-yl)-2-[4-(methylsulfonyl)phenyl]ethanone of formula (I) or of a salt thereof: 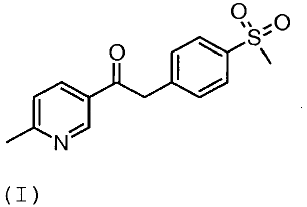 comprising the reaction of (4-methylsolfonyl)phenylacetic acid of formula (IV): 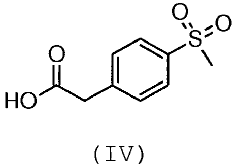 or of alkaline salt of (4-methylsolfonyl) phenylacetic acid of formula (II): 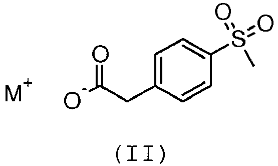 wherein M⁺ is an ion of an alkaline metal,
with the ester of 6-methylpyridine-3-carboxylic acid of formula (III) : 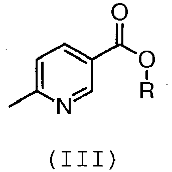 wherein R is a C1-C5 straight o branched alkyl or an aryl or a substituted aryl or a benzyl or a substituted benzyl, in the presence of a Grignard reagent,
**characterized in that** the addition of the two reagents such as the ester of 6-methylpyridine-3-carboxylic acid of formula (III) and the Grignard reagent to the (4-methylsolfonyl) phenylacetic acid of formula (IV) or to the alkaline salt of (4-methylsolfonyl) phenylacetic acid of formula (II) is carried out simultaneously.

2. Process according to claim 1 wherein the alkaline salt of (4-methylsolfonyl) phenylacetic acid of formula (II) is the lithium salt.

3. Process according to anyone of claims from 1 to 2, wherein the ester of 6-methylpyridine-3-carboxylic acid of formula (III) is selected between methyl and ethyl ester.

4. Process according to anyone of claims from 1 to 3 wherein the process is performed with a C1-C5 alkyl Grignard reagent.

5. Process according to claim 4 wherein the Grignard reagent is t-butylmagnesiumchloride.

6. Process according to anyone of claims from 1 to 5 wherein the amount of the Grignard reagent used in case an alkaline salt of the (4-methylsolfonyl)phenylacetic acid of formula (II) is used, is comprised between 1.6 and 1.8 molar equivalents.

7. Process according to claims from 1 to 6 wherein the alkaline salt of (4-methylsolfonyl)phenylacetic acid of formula (II) is prepared from the corresponding acid of formula (IV) and used directly without performing the isolation.

8. The compound alkaline salt of (4-methylsolfonyl)phenylacetic acid of formula (II): 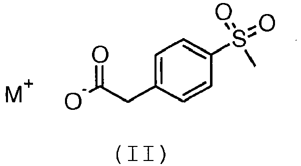 wherein M⁺ is an ion of an alkaline metal, wherein, when M⁺ is Na⁺, the compound is in isolated form.

9. Compound according to claim 8 wherein the metal is lithium, that is to say the compound (II) is the litium (4-methylsolfonyl)phenylacetate.

10. Process for the preparation of the alkaline salt of the (4-methylsolfonyl)phenylacetic acid of formula (II): 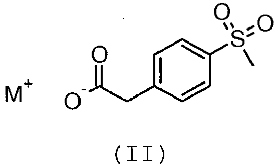 comprising the conversion of (4-methylsolfonyl)phenylacetic acid of formula (IV): 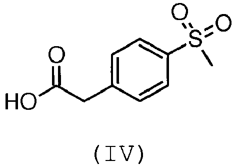 performed by means of a basic salt of an alkaline metal.

11. Process according to claim 10 wherein the basic salt of an alkaline metal is selected from the group consisting in hydroxide, hydride, C1-C5 alkoxy.

12. Use of the compound alkaline salt of (4-methylsolfonyl)phenylacetic acid of formula (II): 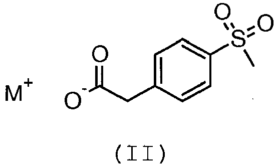 wherein M⁺ is an ion of an alkaline metal, as a reactant for the preparation of 1-(6-methylpyridin-3-yl)-2-[4-(methylsulfonyl)phenyl]ethanone of formula (I) or of a salt thereof:

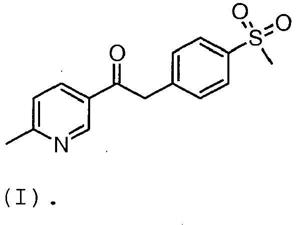

13. Compound 1,3-bis(6-methylpyridin-3-yl)-2-[4-(methylsulfonyl) phenyl]propane-1,3-dione of formula (XII):

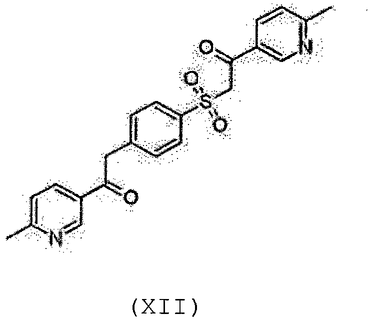

14. Use of the compound of formula (XII) 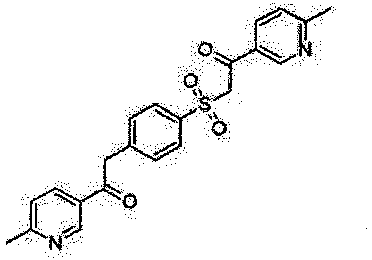 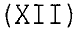 for the identification and the quantification of itself in the compound 1-(6-methylpyridin-3-yl)-2-[4-(methylsulfonyl)phenyl]ethanone of formula (I) 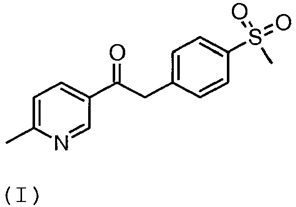 or salt thereof.

## Patentansprüche

1. Verfahren zur Herstellung von 1-(6-Methylpyridin-3-yl)-2-[4-(methylsulfonyl)phenyl]ethanon der Formel (I) oder eines Salzes davon: 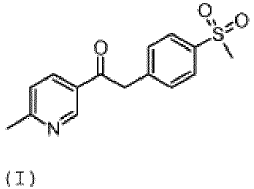 umfassend die Reaktion von (4-Methylsulfonyl)phenylessigsäure der Formel (IV): 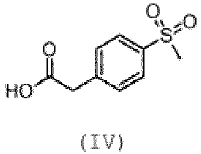 oder von Alkalisalz von (4-Methylsulfonyl)phenylessigsäure der Formel (II): 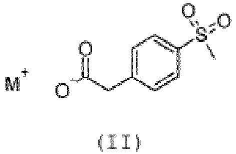 wobei M⁺ ein Ion eines Alkalimetalls ist,
mit dem Ester von 6-Methylpyridin-3-carbonsäure der Formel (III): 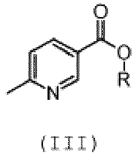 wobei R ein C1-C5 gerades oder verzweigtes Alkyl oder ein Aryl oder ein substituiertes Aryl oder ein Benzyl oder ein substituiertes Benzyl ist, in Gegenwart eines Grignardreagenzes,
**dadurch gekennzeichnet, dass** die Zugabe der zwei Reagenzien, wie dem Ester von 6-Methylpyridin-3-carbonsäure der Formel (III) und dem Grignardreagenz, zu der (4-Methylsulfonyl)phenylessigsäure der Formel (IV) oder zu dem Alkalisalz von (4-Methylsulfonyl)phenylessigsäure der Formel (II) gleichzeitig durchgeführt wird.

2. Verfahren nach Anspruch 1, wobei das Alkalisalz von (4-Methylsulfonyl)phenylessigsäure der Formel (II) das Lithiumsalz ist.

3. Verfahren nach einem der Ansprüche 1 bis 2, wobei der Ester von 6-Methylpyridin-3-carbonsäure der Formel (III) zwischen Methyl- und Ethylester ausgewählt wird.

4. Verfahren nach einem der Ansprüche 1 bis 3, wobei das Verfahren mit einem C1-C5 Alkyl-Grignardreagenz durchgeführt wird.

5. Verfahren nach Anspruch 4, wobei das Grignardreagenz t-Butylmagnesiumchlorid ist.

6. Verfahren nach einem der Ansprüche 1 bis 5, wobei die Menge des verwendeten Grignardreagenzes zwischen 1,6 und 1,8 molaren Äquivalenten umfasst ist, falls ein Alkalisalz der (4-Methylsulfonyl)phenylessigsäure der Formel (II) verwendet wird.

7. Verfahren nach Ansprüchen von 1 bis 6, wobei das Alkalisalz von (4-Methylsulfonyl)phenylessigsäure der Formel (II) von der entsprechenden Säure der Formel (IV) hergestellt wird und direkt verwendet wird, ohne die Isolierung durchzuführen.

8. Alkalisalzverbindung von (4-Methylsulfonyl)phenylessigsäure der Formel (II): 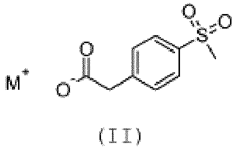 wobei M⁺ ein Ion eines Alkalimetalls ist, worin, wenn M⁺ Na+ ist, ist die Verbindung in isolierter Form.

9. Verbindung nach Anspruch 8, wobei das Metall Lithium ist, nämlich die Verbindung (II) das Lithium(4-methylsulfonyl)phenylacetat ist.

10. Verfahren zur Herstellung des Alkalisalzes der (4-Methylsulfonyl)phenylessigsäure der Formel (II): 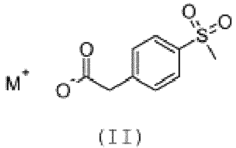 umfassend die Umsetzung von (4-Methylsulfonyl)phenylessigsäure der Formel (IV): 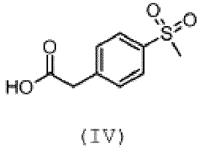 die durch ein basisches Salz eines Alkalimetalls durchgeführt wird.

11. Verfahren nach Anspruch 10, wobei das basische Salz eines Alkalimetalls ausgewählt wird aus der Gruppe bestehend aus Hydroxid, Hydrid, C1-C5-Alkoxy.

12. Verwendung der Alkalisalzverbindung von (4-Methylsulfonyl)phenylessigsäure der Formel (II): 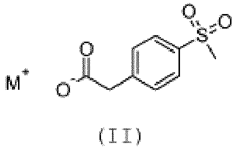 wobei M⁺ ein Ion eines Alkalimetalls ist, als Reaktand, zur Herstellung von 1-(6- Methylpyridin-3-yl)-2-[4-(methylsulfonyl)phenyl]ethanon der Formel (I) oder von einem Salz davon:

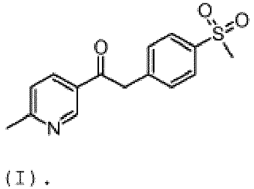

13. Verbindung 1,3-bis(6-Methylpyridin-3-yl)-2-[4-(methylsulfonyl)phenyl]propan-1,3-dion der Formel (XII):

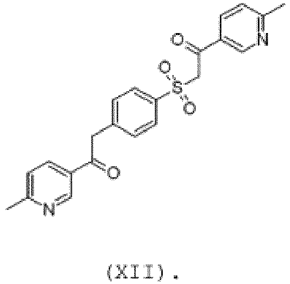

14. Verwendung der Verbindung der Formel (XII) 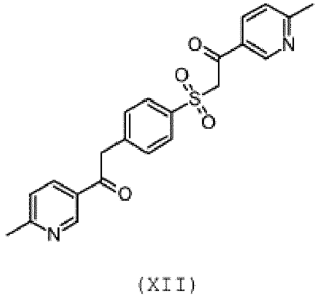 zur Identifizierung und Quantifizierung seiner selbst in der Verbindung 1-(6-Methylpyridin-3-yl)-2-[4-(methylsulfonyl)phenyl]ethanon der Formel (I) 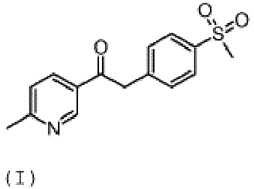 oder Salz davon.

## Revendications

1. Procédé pour la préparation de 1-(6-méthylpyridin-3-yl)-2-[4-(méthylsulfonyl)phényl]éthanone de formule (I) ou d'un sel de celle-ci : 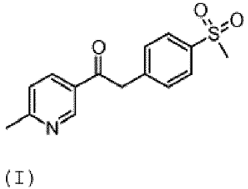 comprenant la réaction d'acide (4-méthylsulfonyl)-phénylacétique de formule (IV) : 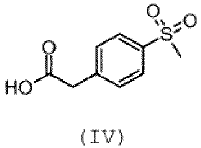 ou d'un sel alcalin d'acide (4-méthylsulfonyl)phénylacétique de formule (II) : 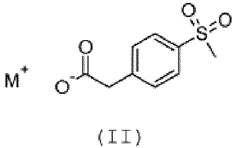 où M⁺ est un ion d'un métal alcalin,
avec l'ester d'acide 6-méthylpyridine-3-carboxylique de formule (III) : 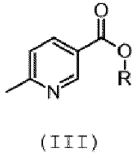 où R est un groupe alkyle linéaire ou ramifié en C1-C5 ou un groupe aryle ou un groupe aryle substitué ou un groupe benzyle ou un groupe benzyle substitué, en présence d'un réactif de Grignard,
**caractérisé en ce que** les additions de deux réactifs, tels que l'ester d'acide 6-méthylpyridine-3-carboxylique de formule (III) et le réactif de Grignard, à l'acide (4-méthylsulfonyl)phénylacétique de formule (IV) ou au sel alcalin d'acide (4-méthylsulfonyl)phénylacétique de formule (II) sont réalisées simultanément.

2. Procédé selon la revendication 1, dans lequel le sel alcalin d'acide (4-méthylsulfonyl)phénylacétique de formule (II) est le sel de lithium.

3. Procédé selon l'une quelconque des revendications 1 à 2, dans lequel l'ester d'acide 6-méthylpyridine-3-carboxylique de formule (III) est choisi parmi un ester méthylique et éthylique.

4. Procédé selon l'une quelconque des revendications 1 à 3, dans lequel le procédé est réalisé avec un réactif de Grignard d'alkyle en C1-C5.

5. Procédé selon la revendication 4, dans lequel le réactif de Grignard est le chlorure de t-butylmagnésium.

6. Procédé selon l'une quelconque des revendications 1 à 5, dans lequel la quantité du réactif de Grignard utilisée dans le cas où un sel alcalin de l'acide (4-méthylsulfonyl)phénylacétique de formule (II) est utilisé, est comprise entre 1,6 et 1,8 équivalents molaires.

7. Procédé selon les revendications 1 à 6, dans lequel le sel alcalin d'acide (4-méthylsulfonyl)phénylacétique de formule (II) est préparé à partir de l'acide correspondant de formule (IV) et est utilisé directement sans réaliser l'isolement.

8. Composé de sel alcalin d'acide (4-méthylsulfonyl)-phénylacétique de formule (II) : 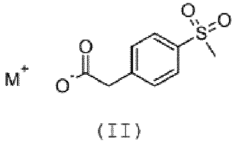 où M⁺ est un ion d'un métal alcalin, où, lorsque M⁺ est Na⁺, le composé est sous une forme isolée.

9. Composé selon la revendication 8, dans lequel le métal est le lithium, c'est-à-dire le composé (II) est le (4-méthylsulfonyl)phénylacétate de lithium.

10. Procédé pour la préparation du sel alcalin de l'acide (4-méthylsulfonyl)phénylacétique de formule (II) : 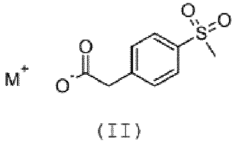 comprenant la conversion d'acide (4-méthylsulfonyl)phényl-acétique de formule (IV) : 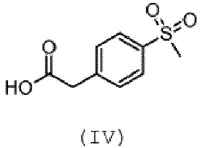 réalisée au moyen d'un sel basique d'un métal alcalin.

11. Procédé selon la revendication 10, dans lequel le sel basique d'un métal alcalin est choisi dans le groupe constitué d'un hydroxyde, d'un hydrure, d'un groupe alcoxy en C1-C5.

12. Utilisation du composé de sel acalin d'acide (4-méthylsulfonyl)phénylacétique de formule (II) : 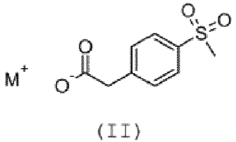 où M⁺ est un ion d'un métal alcalin, en tant que réactif, pour la préparation de 1-(6-méthylpyridin-3-yl)-2-[4-(méthylsulfonyl)phényl]éthanone de formule (I) ou d'un sel de celle-ci :

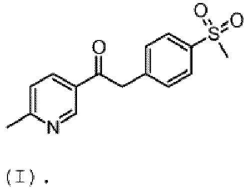

13. Composé de 1,3-bis(6-méthylpyridin-3-yl)-2-[4-(méthylsulfonyl)phényl]propane-1,3-dione de formule (XII) :

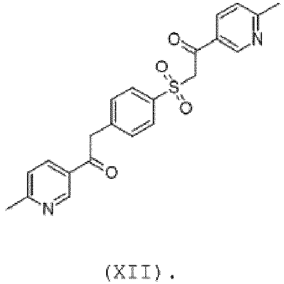

14. Utilisation du composé de formule (XII) 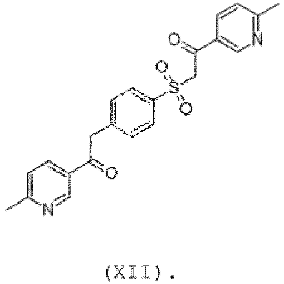 pour l'identification et la quantification de lui-même dans le composé de 1-(6-méthylpyridin-3-yl)-2-[4-(méthylsulfonyl)phényl]-éthanone de formule (I) 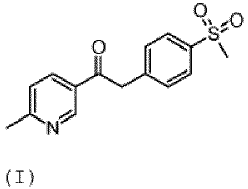 ou d'un sel de celle-ci.
